# EUROPEAN PATENT SPECIFICATION

(11) **EP 3 331 506 B1**
(45) Date of publication and mention of the grant of the patent: **20.03.2019**
(21) Application number: 16770468.3
(22) Date of filing: 16.09.2016
(51) Int. Cl.: A61K 9/26, A61K 9/50, A61K 9/48, A61K 31/575, A61P 1/16

(54) **PEDIATRIC FORMULATION COMPRISING BILE ACID**
PÄDIATRISCHE FORMULIERUNG ENTHALTEND GALLENSÄURE
FORMULATION PÉDIATRIQUE COMPRENANT DE L'ACIDE BILIAIRE

(30) Priority: 16.09.2015 EP 15185472; 16.09.2015 US 201514855698
(43) Date of publication of application: 13.06.2018
(73) Proprietor: Laboratoires C.T.R.S., 92100 Boulogne-Billancourt (FR)
(72) Inventor: DESCHAMPS, Bernard, 16250 Champagne-Vigny (FR); FERRY, Antoine, 75007 Paris (FR); VOGEL, Andreas, 92310 Sèvres (FR)
(74) Representative: Icosa
(86) International application number: PCT/EP2016/071920
(87) International publication number: WO 2017/046297

(56) References cited:
- US-A- 5 405 621

## Description

### FIELD OF INVENTION

The present invention relates to pediatric drug formulation. Especially, this invention relates to the manufacturing of granules comprising a core comprising a drug having an unpleasant taste, which may be primary bile acid, such as for example cholic acid, said core being coated with a taste-masking coating. The purpose of the invention is to fully mask the unpleasant taste of the primary bile acid, while ensuring its availability at the duodenum biological site. The granules of the invention can be further formulated in a formulation which can be a dosage form, including either sprinkle capsule, dispersible tablet, orodispersible tablet, non-aqueous liquid formulation, or sachet.

### BACKGROUND OF INVENTION

Bile acids are steroid acids found predominantly in the bile of mammals and others vertebrates. Bile acids comprise primary bile acids and secondary bile acids. The primary bile acids are cholic acid and chenodeoxycholic acid. Main secondary bile acids are: deoxycholic acid, lithocholic acid, and ursodeoxycholic acid.

Primary bile acids synthesized by the liver, are generally stored in the gallbladder, and discharged into the duodenum, where they emulsify dietary fats to allow their absorption. Secondary bile acids result from bacterial action of intestinal bacteria on primary acids, predominantly in the colon. Primary and secondary bile acids undergo enterohepatic circulation after absorption which occurs for the most part in the ileum.

It is known in the prior art that bile acids administered to mammals can remedy bile acid deficiencies caused by various diseased conditions of the liver, such as gallstones, liver toxicity due to metabolites, drug related toxicity, colon cancer and deficiency associated with poor digestion of fats and lipids in the intestine.

Cholic acid is well-known for the treatment of inborn errors in primary bile acid synthesis in infants, children and adolescents. These deficiencies in primary bile acids synthesis lead to hepatic failure and/or to progressive neurological impairment. In some countries, cholic acid is also authorized for the treatment of peroxisomal disorders such as Zellweger spectrum disorders.

However, a major drawback in the therapeutic use of bile acids is that bile acids required the presence of certain conditions in order for them to be safe and effective. Another major drawback in the therapeutic use of bile acids, especially cholic acid, is that bile acids are very bitter and this most unpleasant taste cannot be adequately masked by the mere addition of sweeteners and/or flavoring agents.

US 5,405,621 discloses buffer-stabilized secondary bile acid compositions, specifically ursodeoxycholic acid compositions, for ingestion by a mammal, these compositions addressing the following issues: taste-masking and a sustained release in the intestine. In order to bring a solution to these issues, ursodeoxycholic acid is administered as a microsphere of buffer-stabilized ursodeoxycholic acid associated with an acid-resistant coating. Indeed, when ursodeoxycholic acid is administered exogenously to the patient, the conditions in the stomach, namely the presence of acid, render ursodeoxycholic acid insoluble and diminish its availability. The formulation of US 5,405,621 brings a solution by allowing, through its coating, delayed disintegration and sustained-release of the buffer-stabilized bile acid into the intestine, far after the duodenum, at the site of absorption.

However, US 5,405,621 does not address the issue of providing a bile acid which has to be released in the stomach and made bioavailable in the duodenum, and the microspheres disclosed in US 5,405,621 are not suitable or applicable in any way to primary bile acid therapy for deficiencies of primary bile acid synthesis. Moreover, a release of primary bile acid after the stomach would induce excess concentration of bile acids in the colon and would cause undesirable side effect such as diarrhea.

Cholic acid formulations currently commercially available are in the form of capsules; they are meant and conceived to be swallowed all at once, and consequently these formulations cannot be used in pediatric treatment or for treating patients having swallowing problems. For babies and children who cannot swallow capsules, the pediatric treatment currently used is to add the contents of the capsule to milk, including expressed breast milk, juice or fruit puree, exposing these patients, especially baby patients, to the unpleasant taste of cholic acid and refusal of food mixed with the therapeutic agent.

Therefore, primary bile acid formulations, especially cholic acid formulations, for oral administration fully masking the unpleasant taste, and having an acid dissolution profile as efficient as the existing swallowable form Orphacol®, and making the acid available for physiological action in the duodenum and suitable for administration in pediatric treatment or to patients having swallowing difficulties or swallowing disability, is currently an unmet need.

### SUMMARY

This invention brings a solution to this unmet need, using two main ingredients: at least one unbuffered primary bile acid and a gastric-fluid soluble taste-masking coating fully surrounding the primary bile acid. Also, the Applicant took in high consideration the needs of the populations of interested patients, especially baby patients, and designed a formulation with the idea of being the simplest possible formulation ever, i.e. encompassing the smallest possible number of ingredients and notably excluding excipients with possible harmful effects, such as preservatives.

This invention thus relates to a granule comprising a core, this core comprising at least one unbuffered primary bile acid and at least one binder; and a coating surrounding the core, this coating comprising at least one gastric-fluid-soluble-and-taste-masking compound. According to one embodiment, the primary bile acid is cholic acid. According to one embodiment, the core of said granule further comprises at least one excipient, herein referred to as Excipient A, preferably selected from sugars, polyols, calcium salts and polymers.

According to one embodiment, the at least one binder, is selected from natural polymers, synthetic polymers, or sugars.

According to one embodiment, the at least one gastric-fluid-soluble-and-taste-masking compound is selected from starches, polyvinylpyrrolidones, gelatin, methylcellulose, hydroxyethylcellulose, hydroxypropylcellulose, hydroxypropyl methyl cellulose, microcrystalline cellulose, methacrylic acid copolymer and combinations thereof.

According to one embodiment, the amount of unbuffered primary bile acid ranges from 1% w/w to 90% w/w, preferably from 5% to 80% w/w, more preferably from 5% w/w to 60% w/w, even more preferably from 10% w/w to 40% w/w in weight to the total weight of said granule.

According to one embodiment, the amount of said a binder ranges from more than 0% w/w to 30% w/w, preferably from more than 0% w/w to 20% w/w, more preferably from 1% w/w to 10% w/w, in weight to the total weight of said granule. According to one embodiment, the amount of said taste-masking compound ranges from 1% w/w to 90% w/w, preferably from 5% w/w to 50% w/w, more preferably from 10% w/w to 40% w/w, in weight to the total weight of said granule. According to one embodiment, the amount of said excipient A ranges from 0% w/w to 70% w/w, preferably from 0% w/w to 60% w/w, more preferably from 0% w/w to 40% w/w, in weight to the total weight of said granule.

According to one embodiment, the granule of the invention, comprises:
- from 70% to 80% in weight relative to the total weight of the formulation of cholic acid;
- from 1% to 10% in weight relative to the total weight of the formulation of polyvinylpyrrolidone;
- from 10% to 40% in weight relative to the total weight of the formulation of Poly(butyl methacrylate-co-(2-dimethylaminoethyl) methacrylate-co-methyl methacrylate) (1:2:1).

According to another embodiment, the granule of the invention, comprises:
- from 10% to 20% in weight relative to the total weight of the formulation of cholic acid;
- from 50% to 65% in weight relative to the total weight of the formulation of dicalcium phosphate;
- from 2.5% to 12.5% in weight relative to the total weight of the formulation of polyvinylpyrrolidone;
- from 12.5% to 22.5% in weight relative to the total weight of the formulation of Poly(butyl methacrylate-co-(2-dimethylaminoethyl) methacrylate-co-methyl methacrylate) (1:2:1).

This invention also relates to a formulation comprising at least one granule of the invention and at least one excipient herein referred to as Excipient B, which is preferably a diluent, a disintegrant, and/or a lubricant. According to one embodiment, the amount of at least one unbuffered primary bile acid present in the granule ranges from 1% w/w to 90% w/w, preferably from 5% w/w to 60% w/w, more preferably from 10% w/w to 40% w/w in weight to the total weight of the formulation. According to one embodiment of the formulation, the amount of said gastric-fluid-soluble-and-taste-masking compound ranges from 1% w/w to 90% w/w, preferably from 5% w/w to 50% w/w, more preferably from 10% w/w to 40% w/w, in weight to the total weight of the formulation. According to one embodiment of this formulation, the amount of said excipient B ranging from more than 0% w/w to 90% w/w, preferably from 5% w/w to 70% w/w, more preferably from 10% w/w to 60% w/w, in weight to the total weight of the formulation. According to one embodiment, the particle size distribution of the granules in the formulation ranges from 30 µm to 1 000 µm, preferably from 30 µm to 710 µm, more preferably from 90 µm to 500 µm, even more preferably from 125 µm to 355 µm. According to one embodiment, the particle size distribution of said granule ranges from 30 µm to 1 000 µm, preferably from 30 µm to 710 µm, more preferably from 90 µm to 500 µm, even more preferably from 125 µm to 500 µm.

According to one embodiment, the formulation comprises:
- from 10% to 20% in weight relative to the total weight of the formulation of cholic acid;
- from 1% to 12% in weight relative to the total weight of the formulation of Mannitol;
- from 1% to 12% in weight relative to the total weight of the formulation of Microcrystalline cellulose;
- from 0.01% to 1.5% in weight relative to the total weight of the formulation of hydroxylpropylcellulose;
- from 8% to 18% in weight relative to the total weight of the formulation of Poly(butyl methacrylate-co-(2-dimethylaminoethyl) methacrylate-co-methyl methacrylate) (1:2:1);
- from 43% to 53% in weight relative to the total weight of the formulation of microcrystalline cellulose;
- from 0% to 10% in weight relative to the total weight of the formulation of sodium croscarmellose;
- from 0% to 10% in weight relative to the total weight of the formulation of magnesium stearate.

This invention also relates to a process for manufacturing at least one granule of the invention comprising mixing at least one unbuffered primary bile acid with at least one binder; granulating the said at least one unbuffered primary bile acid with at least one binder using a wet granulation and drying the obtained granules; and then coating the said granules with at least one gastric-fluid-soluble-and-taste-masking compound.

This invention also relates to a process for manufacturing a formulation of the invention comprising mixing at least one unbuffered primary bile acid with at least one binder; granulating the said at least one unbuffered primary bile acid with at least one binder using a wet granulation and drying the obtained granules; then coating the said granules with at least one gastric-fluid-soluble-and-taste-masking compound, and then mixing at least one unbuffered primary bile acid with at least one excipient, referred to as Excipient B.

According to one embodiment, the process for the manufacturing a formulation of the invention consists of:
a) Mixing at least one unbuffered primary bile acid, especially unbuffered cholic acid with at least one excipient A;
b) Granulating the said at least one unbuffered primary bile acid, especially unbuffered cholic acid with the at least one excipient A using a wet granulation in presence of at least one binder;
c) Drying the said granules containing at least one unbuffered primary bile acid granules, especially unbuffered cholic acid granules;
d) Calibrating the said at least one unbuffered primary bile acid granules, especially unbuffered cholic acid granules;
e) Coating the said at least one unbuffered primary bile acid granules, especially unbuffered cholic acid granules with at least one gastric-fluid-soluble-and-taste-masking compound;
f) Drying the said coated at least one unbuffered primary bile acid granules, especially coated, unbuffered cholic acid granules;
g) Calibrating the said coated at least one unbuffered primary bile acid granules, especially coated unbuffered cholic acid granules;
h) Mixing the said coated at least one unbuffered primary bile acid granules, especially unbuffered cholic acid granules with at least one excipient B; and
i) Tableting the final blend to obtain dispersible or orodispersible tablets or filling the final blend in sprinkle capsules or sachets.

The present invention also relates to a pharmaceutical composition comprising at least one granule of the invention or a formulation of the invention. The present invention further relates to a medicament comprising at least one granule of the invention or a formulation of the invention.

In another aspect, the present invention relates to a granule of the invention or a formulation of the invention or a pharmaceutical composition of the invention comprising an effective amount of primary bile acid for use in the treatment of a patient suffering from a deficiency in primary bile acid synthesis.

This invention also relates to a unit dosage form comprising a container containing at least one granule or a formulation of the invention.

### DEFINITIONS

In the present invention, the following terms have the following meanings:
- **"Binder"** refers to pharmaceutical glue dissolved in water or solvent and included in the wet granulation process. The binder forms a bond with the powders during the granulation process.
- **"Core"** refers to the central or innermost part.
- **"Eudragit**®**-type polymer"** refers to poly(meth)acrylates for pharmaceutical applications described in Ph.Eur as Methacrylic Acid - Ethyl Acrylate Copolymer, Methacrylic Acid - Ethyl Acrylate Copolymer dispersion, Methacrylic Acid - Methyl Methacrylate Copolymer, Basic Butylated Methacrylate Copolymer, Ammonio Methacrylate Copolymer, Polyacrylate Dispersion.
- **"Excipient"** refers to natural or synthetic substance formulated alongside the active ingredient of a medication included for the purpose of bulking up formulations that contain potent active ingredients (thus often referred to as "bulking agents," "fillers," or "diluents"), or to confer a therapeutic enhancement on the active ingredient in the final dosage form, such as facilitating drug absorption or solubility.
- **"gastric-fluid-soluble taste-making compound"** refers to a taste-making compound which is soluble at the pH of the gastric fluid.
- **"Granulation"** refers to the act or process in which primary powder particles are made to adhere to form larger, multiparticle entities called granules.
- **"Granule"** refers to multiparticle entities.
- **"Primary bile acid"** refers to cholic acid and chenodeoxycholic acid.
- **"Taste-masking compound"** refers to a compound used to mask the bitter and/or unpleasant taste of the active ingredient.
- **"unbuffered primary bile acid"** refers to a primary bile acid which is not neutralized by a buffer salt.

### DETAILED DESCRIPTION

In a first aspect, the present invention relates to a granule comprising: (1) a core comprising (i) at least one unbuffered primary bile acid, especially unbuffered cholic acid and (ii) at least one binder; and (2) a coating fully surrounding the core, said coating comprising at least one gastric-fluid-soluble-and-taste-masking compound. According to one embodiment, the core further comprises at least one excipient (Excipient A).

According to one embodiment, the granule of the invention comprises or consists of: (1) a core consisting of (i) at least one unbuffered primary bile acid, especially unbuffered cholic acid, (ii) one binder and optionally (iii) at least one excipient (Excipient A); and (2) a coating consisting of one gastric-fluid-soluble-and-taste-masking compound.

According to a first embodiment, the core of the granule comprises at least one unbuffered primary bile acid, especially unbuffered cholic acid and at least one binder, preferably selected from natural polymer, synthetic polymer or sugar.

According to a second embodiment, the core of said granule comprises at least one at least one unbuffered primary bile acid, especially at least one unbuffered cholic acid and at least one binder, preferably selected from natural polymer, synthetic polymer or sugar and at least one excipient (Excipient A) preferably selected from sugars, polyols, calcium salts and polymers.

According to third embodiment, the granule of the invention consists of: (1) a core consisting of (i) at least one unbuffered primary bile acid, especially unbuffered cholic acid, (ii) one binder and (iii) at least one excipient (Excipient A); and (2) a coating consisting of one gastric-fluid-soluble-and-taste-masking compound.

According to one embodiment, at least one unbuffered primary bile acid is selected from unbuffered cholic acid and unbuffered chenodeoxycholic acid. In a preferred embodiment, unbuffered primary bile acid is unbuffered cholic acid.

According to one embodiment the at least one binder may be selected from natural polymer, synthetic polymer or sugar. According to one embodiment, the at least one binder is selected from starch, pregelatinized starch, gelatin, acacia, alginic acid, sodium alginate, polyvinylpyrrolidone (PVP), methylcellulose, hydroxylpropylcellulose (HPC), hypromellose (HPMC), sodium carboxymethylcellulose (Na-CMC), ethylcellulose, glucose, sucrose, sorbitol and combinations thereof. According to one embodiment, the binder is selected from polyvinylpyrrolidone (PVP) or hydroxylpropylcellulose (HPC). According to one embodiment, the binder is polyvinylpyrrolidone (PVP). According to one embodiment, the binder is hydroxylpropylcellulose (HPC).

According to one embodiment, the at least one excipient A is selected from sugars, polyols, calcium salts and polymers. According to one embodiment, the excipient A or combinations of excipients A are selected from sucrose, lactose, maltose, lactulose, trehalose, cellobiose, melibiose, isomaltose, glycerol, erythriol, xylitol, arabitol, ribitol, sorbitol, dulcitol, mannitol, volemitol, maltitol, isomaltitol, lactitol, calcium carbonate, dicalcium phosphate, tricalcium phosphate, microcrystalline cellulose and combinations thereof. According to one embodiment, the excipient A or combinations of excipients A is selected from microcrystalline cellulose, mannitol, and dicalcium phosphate. According to one embodiment, the excipient A or combinations of excipients A is a combination of microcrystalline cellulose and mannitol. According to one embodiment, the excipient A is dicalcium phosphate.

According to one embodiment gastric-fluid-soluble-and-taste-masking compound is selected from starches, polyvinylpyrrolidones, gelatin, methylcellulose, hydroxyethylcellulose, hydroxypropylcellulose, hydroxypropyl methyl cellulose, microcrystalline cellulose and methacrylic acid copolymer and combinations thereof. According to one embodiment, the at least one gastric-fluid-soluble-and-taste-masking compound is selected from Eudragit®-type polymers (Evonik Industries AG, Darmstadt, Germany), sodium carboxymethylcellulose (Na-CMC) polyvinylpyrrolidone, hydroxylethylcellulose, hydroxypropylcellulose, hydroxypropyl methyl cellulose, microcrystalline cellulose polyvinyl acetate, polycarbophil, polyacrylic acid, cellulose ethers, cellulose ester, polyvinyl acetate, methyl acrylate, hydroxyl ethyl methacrylate, vinyl pyridine, and combinations thereof. According to one embodiment, the at least one gastric-fluid-soluble-and-taste-masking compound is preferably selected from Eudragit® E-type polymers, more preferably selected from Eudragit® E PO, Eudragit® E100 or Eudragit® E12.5, even more preferably selected from Eudragit® E PO. According to one embodiment, the at least one gastric-fluid-soluble-and-taste-masking compound is preferably selected from Basic Butylated Methacrylate Copolymer or Ammonio Methacrylate Copolymer, more preferably selected from Poly(butyl methacrylate-co-(2- demethylaminoeethyl) methacrylate-co-methyl methacrylate) 1:2:1, Poly(butyl methacylate-co-(2-dimethylaminoethyl) methacrylate-co-methyl methacrylate) 1:2:1 or Poly(butyl methacrylate-co-(2-dimethylaminoethyl) methacrylate-co-methyl methacrylate) (1:2:1). According to a preferred embodiment, the at least one gastric-fluid-soluble-and-taste-masking compound is Eudragit® E PO (Poly(butyl methacrylate-co-(2-dimethylaminoethyl) methacrylate-co-methyl methacrylate) (1:2:1)).

According to one embodiment, the granule of the invention, comprises:
- cholic acid;
- polyvinylpyrrolidone;
- Poly(butyl methacrylate-co-(2-dimethylaminoethyl) methacrylate-co-methyl methacrylate) (1:2:1).

According to another embodiment, the granule of the invention, comprises:
- cholic acid;
- dicalcium phosphate;
- polyvinylpyrrolidone;
- Poly(butyl methacrylate-co-(2-dimethylaminoethyl) methacrylate-co-methyl methacrylate) (1:2:1).

According to another embodiment, the granule of the invention, comprises:
- cholic acid;
- mannitol;
- microcrystalline cellulose;
- hydroxylpropylcellulose;
- Poly(butyl methacrylate-co-(2-dimethylaminoethyl) methacrylate-co-methyl methacrylate) (1:2:1).

According to one embodiment, the particle size distribution of said granule ranges from 30 µm to 1 000 µm, preferably from 30 µm to 710 µm, more preferably from 90 µm to 500 µm, even more preferably from 125 µm to 500 µm.

According to one embodiment, the amount of unbuffered primary bile acid ranges from 1% w/w to 90% w/w, preferably from 5% to 80% w/w, more preferably from 5% w/w to 60% w/w, even more preferably from 10% w/w to 40% w/w in weight to the total weight of said granule.

According to a preferred embodiment, the amount of unbuffered primary bile acid ranges from 1% w/w to 90% w/w, preferably from 5% to 80% w/w, more preferably from 50% to 80% w/w in weight to the total weight of said granule when the granule consists of a core consisting of a unbuffered primary bile acid and a binder, and a coating.

According to a another preferred embodiment, the amount of unbuffered primary bile acid ranges from 1% w/w to 90% w/w, more preferably from 5% w/w to 60% w/w, even more preferably from 10% w/w to 40% w/w in weight to the total weight of said granule when the granule consists of a core consisting of a unbuffered primary bile acid, an excipient and a binder, and a coating.

According to one embodiment, the amount of said a binder ranges from more than 0% w/w to 30% w/w, preferably from more than 0% w/w to 20% w/w, more preferably from 1% w/w to 10% w/w, in weight to the total weight of said granule.

According to one embodiment, the amount of said taste-masking compound ranges from 1% w/w to 90% w/w, preferably from 5% w/w to 50% w/w, more preferably from 10% w/w to 40% w/w, in weight to the total weight of said granule.

According to one embodiment, the amount of said excipient A ranges from 0% w/w to 70% w/w, preferably from 0% w/w to 60% w/w, more preferably from 0% w/w to 40% w/w, in weight to the total weight of said granule.

According to the invention, the gastric-fluid-soluble-and-taste-masking coating does not solubilize upon contact of the granules of the invention with the oral cavity (pH 6.5 to 7.4), and prevents the release of primary bile acid, especially cholic acid, therefore avoiding any perception of bitterness. At the time of arrival in the stomach, the pH between 1 and 5 solubilizes the coating thereby releasing the primary bile acid.

It is readily understandable that this invention, in a second aspect, also relates to a formulation comprising at least one unbuffered primary bile acid, especially cholic acid, for oral administration, wherein the unpleasant taste of primary bile acid, especially of cholic acid, is fully masked, i.e. not perceptible in the mouth. In one embodiment, the formulation encompasses at least one granule as described here above. According to one embodiment, the formulation comprises or consists of a composition comprising granules of the invention and at least one excipient B.

According to one embodiment, the at least one excipient B is selected from at least one diluent, at least one disintegrant and at least one lubricant or a combination thereof. According to one embodiment, the at least one excipient B is selected from sugars, polyols, calcium salts, natural polymer, synthetic polymer, hydrophobic or hydrophilic lubricants.

According to one embodiment, the at least one diluent is selected from sugars, polyols, calcium salts and polymers. According to one embodiment, the at least one diluent is selected from sucrose, lactose, maltose, lactulose, trehalose, cellobiose, melibiose, isomaltose, glycerol, erythriol, xylitol, arabitol, ribitol, sorbitol, dulcitol, mannitol, volemitol, maltitol, isomaltitol, lactitol, calcium carbonate, dicalcium phosphate, tricalcium phosphate, microcrystalline cellulose and combinations thereof, preferably is selected from microcrystalline cellulose, maltitol, and combinations thereof. According to a first embodiment, the diluent is microcrystalline cellulose. According to a second embodiment, the diluent is maltitol.

According to one embodiment, the at least one disintegrant is selected from natural polymer or synthetic polymer. According to one embodiment, the at least one disintegrant is selected from povidone, crospovidone, microcrystalline cellulose, sodium croscarmellose, methylcellulose, starch, pregelatinized starch, carboxymethyl starch, alginic acid, sodium alginate. According to one embodiment, the disintegrant is preferably crospovidone or sodium croscarmellose. According to one embodiment, the disintegrant is preferably sodium croscarmellose.

According to one embodiment, the at least one lubricant is selected from hydrophobic or hydrophilic lubricants. According to one embodiment, the at least one lubricant is selected from stearic acid, magnesium stearate, sodium stearyl fumarate. According to one embodiment, the at least one lubricant is preferably sodium stearyl fumarate or magnesium stearate. According to one embodiment, the at least one lubricant is magnesium stearate. According to one embodiment, the at least one lubricant is sodium stearyl fumarate.

According to one embodiment, the at least one excipient B is a combination of microcrystalline cellulose, sodium croscarmellose and magnesium stearate.

According to one embodiment, the formulation comprises:
- cholic acid;
- Mannitol;
- Microcrystalline cellulose;
- hydroxylpropylcellulose;
- Poly(butyl methacrylate-co-(2-dimethylaminoethyl) methacrylate-co-methyl methacrylate) (1:2:1);
- microcrystalline cellulose;
- sodium croscarmellose;
- magnesium stearate.

According to another embodiment, the formulation comprises:
- cholic acid;
- Mannitol;
- Microcrystalline cellulose;
- hydroxylpropylcellulose;
- Poly(butyl methacrylate-co-(2-dimethylaminoethyl) methacrylate-co-methyl methacrylate) (1:2:1);
- microcrystalline cellulose;
- sodium croscarmellose;
- sodium stearyl fumarate.

According to one embodiment, the amount of at least one unbuffered primary bile acid, especially unbuffered cholic acid, ranges from 1% w/w to 90% w/w, preferably from 5% w/w to 60% w/w, more preferably from 10% w/w to 40% w/w in weight to the total weight of the formulation.

According to one embodiment, the amount of said binder ranges from more than 0% w/w to 30% w/w, preferably from more than 0% w/w to 20% w/w, more preferably from more than 0.5% w/w to 10% w/w, in weight to the total weight of the formulation.

According to one embodiment, the amount of said taste-masking compound ranges from 1% w/w to 90% w/w, preferably from 2% w/w to 50% w/w, more preferably from 2% w/w to 25% w/w in weight to the total weight of the formulation.

According to one embodiment, the amount of said excipient A ranging from 0% w/w to 90% w/w, preferably from 5% w/w to 80% w/w, more preferably from 5% w/w to 70% w/w, even more preferably from 10% w/w to 60% w/w, in weight to the total weight of the formulation.

According to one embodiment, the amount of said excipient B ranging from more than 0% w/w to 90% w/w, preferably from 5% w/w to 70% w/w, more preferably from 10% w/w to 60% w/w, in weight to the total weight of the formulation.

According to one embodiment, the amount of said diluent ranging from 0% w/w to 90% w/w, preferably from 5% w/w to 70% w/w, more preferably from 10% w/w to 60% w/w, in weight to the total weight of the formulation.

According to one embodiment, the amount of said disintegrant ranging from 0% w/w to 15% w/w, preferably from 2% w/w to 12% w/w, more preferably from 2% w/w to 10% w/w, in weight to the total weight of the formulation.

According to one embodiment, the amount of said lubricant ranging from 0% w/w to 10% w/w, preferably from 0.1% w/w to 5% w/w, more preferably from 0.25% w/w to 2% w/w, in weight to the total weight of the formulation.

In a third aspect, the present invention relates to a process for manufacturing at least one granule of the invention comprising at least one binder wherein the steps are carried out in the following order:
a1. Mixing at least one unbuffered primary bile acid;
b1. Granulating the said at least one unbuffered primary bile acid with the at least one binder;
c1. Coating the said granules with at least one gastric-fluid-soluble-and-taste-masking compound.

According to a first embodiment, steps of the said process for manufacturing at least one granule of the invention comprising at least one excipient A and at least one binder are carried out in the following order:
a2. Mixing at least one unbuffered primary bile acid with at least one excipient A and at least one binder;
b2. Granulating the said at least one unbuffered primary bile acid with the at least one excipient A and the at least one binder;
c2. Coating the said granules with at least one gastric-fluid-soluble-and-taste-masking compound.

According to one embodiment, the granules obtained after the granulating step have a particle size distribution ranging from 10 µm to 710 µm, preferably from 10 µm to 500 µm, more preferably from 30 µm to 355 µm, even more preferably from 125 µm to 250 µm.

In a fourth aspect, the present invention relates a process for manufacturing a formulation of the invention comprising the following steps:
- Mixing at least one unbuffered primary bile acid with at least one binder and optionally at least one excipient A;
- Granulating the said at least one unbuffered primary bile acid the at least one binder and optionally with the at least one excipient A;
- Coating the said granules with at least one gastric-fluid-soluble-and-taste-masking compound;
- Mixing coated at least one unbuffered primary bile acid granules, with at least one excipient B.

According to a first embodiment, steps of the said process for manufacturing a formulation of the invention comprising at least one granule comprising at least one binder are carried out in the following order:
a3. Mixing at least one unbuffered primary bile acid;
b3. Granulating the said at least one unbuffered primary bile acid with the at least one binder;
c3. Coating the said granules with at least one gastric-fluid-soluble-and-taste-masking compound;
d3. Mixing coated at least one unbuffered primary bile acid granules, with at least one excipient B.

According to a second embodiment, steps of the said process for manufacturing a formulation of the invention comprising at least one granule further comprising at least one excipient A are carried out in the following order:
a4. Mixing at least one unbuffered primary bile acid with at least one excipient A and at least one binder;
b4. Granulating the said at least one unbuffered primary bile acid with the at least one excipient A and the at least one binder;
c4. Coating the said granules with at least one gastric-fluid-soluble-and-taste-masking compound;
d4. Mixing coated at least one unbuffered primary bile acid granules, with at least one excipient B.

According to one embodiment, the process of the invention can further comprise a step of calibration of the at least one unbuffered primary bile acid granules before coating.

According to one embodiment, the process of the invention can further comprise a step of drying of the coated at least one unbuffered primary bile acid granules after coating.

According to one embodiment, the process of the invention can further comprise a step of calibration of the at least one unbuffered primary bile acid granules after coating. According to one embodiment, the calibration of the at least one unbuffered primary bile acid granules after coating is performed after the step of drying.

According to one embodiment, the process of the invention can further comprise a final step of tableting the final blend to obtain dispersible or orodispersible tablets or filling the final blend in sprinkle capsules or sachets.

According to a preferred embodiment, the process for the manufacturing a formulation of the invention consists of:
a5. Mixing at least one unbuffered primary bile acid, especially unbuffered cholic acid with at least one excipient A;
b5. Granulating the said at least one unbuffered primary bile acid, especially unbuffered cholic acid with the at least one excipient A using a wet granulation in presence of at least one binder;
c5. Drying the said granule containing at least one unbuffered primary bile acid granules, especially unbuffered cholic acid granules;
d5. Calibrating the said at least one unbuffered primary bile acid granules, especially unbuffered cholic acid granules;
e5. Coating the said at least one unbuffered primary bile acid granules, especially unbuffered cholic acid granules with at least one gastric-fluid-soluble-and-taste-masking compound;
f5. Drying the said coated at least one unbuffered primary bile acid granules, especially coated unbuffered cholic acid granules;
g5. Calibrating the said coated at least one unbuffered primary bile acid granules, especially coated unbuffered cholic acid granules;
h5. Mixing the said coated at least one unbuffered primary bile acid granules, especially unbuffered cholic acid granules with at least one excipient B; and
i5. Tableting the final blend to obtain dispersible or orodispersible tablets or filling the final blend in sprinkle capsules or sachets.

According to one embodiment, granulation can be a dry granulation performed with a swaying granulator (e.g. High Efficiency Swaying Granulator manufactured by Changzhou Kewei Mechanical Manufacturing Co., Ltd) or a high shear mixer-granulator (e.g. Aeromatic-Fielder™ manufactured by GEA Pharma Systems), or a wet granulation performed with air in a fluidized bed dryer granulator (e.g. GPCG1 Top Spray manufactured by Glatt) or an impeller in a high shear granulator (e.g. VG25 manufactured by Glatt), or screws in a twin screw granulator (e.g. Pharma 24 TSG Twin Screw Granulator manufactured by Thermo Scientific).

According to one embodiment, the granulation is a wet granulation. The liquid solution used for the wet granulation can be aqueous based or solvent based. Typical liquids include water, ethanol, isopropanol and combinations thereof.

According to one embodiment, the wet granulation is performed with a high shear impeller or a fluidized bed dryer granulator.

According to one embodiment, the wet granulation is performed with a high shear impeller using an aqueous liquid solution.

According to one embodiment, the wet granules are discharged and dried in a fluidized bed dryer. According to one embodiment, the wet granules are dried in a fluidized bed dryer at a temperature lower than 60°C, preferably lower than 46°C.

According to one embodiment, coating can be performed in a fluidized bed dryer granulator (e.g. GPCG1 Wurster manufactured by Glatt) using aqueous based or solvent based liquid solution; more preferably coating is performed in a fluidized bed dryer granulator using aqueous based liquid solution.

In a fifth aspect, the present invention relates to a pharmaceutical composition comprising at least one granule of the invention and at least one pharmaceutically acceptable carrier.

In a sixth aspect, the present invention relates to a medicament comprising at least one granule of the invention or the pharmaceutical composition of the invention as active ingredient.

In a seventh aspect, the present invention relates to a unit dosage form comprising a container containing at least one granule of the invention. In one embodiment, the unit dosage form comprises a container containing a formulation of the invention. Generally, such unit dosages will contain between 10 and 1000 mg, and usually between 10 and 250 mg, preferably between 10 and 50 mg of cholic acid, e.g. about 10 mg, 25 mg, 50 mg per unit dosage.

In an eighth aspect, the present invention relates to a pharmaceutical composition comprising an effective amount of primary bile acid, especially cholic acid for use in the treatment of a patient suffering from a deficiency in primary bile acid synthesis.

According to one embodiment, the patient is a human.

According to a first embodiment, the patient is aged 1 month to 6 years.

According to a second embodiment, the patient of any age has swallowing difficulty or swallowing disability.

### EXAMPLES

The present invention is further illustrated by the following examples.

### Example 1: Manufacturing of the cholic acid pediatric formulation with granulation, coating then mixing with an excipient.

### Formulation 1

The following table presents the formulation of the cholic acid pediatric formulation.

| **Chemical name ingredient** | **Dosage unit (mg)** | **Quantity %(w/w)** |
|---|---|---|
| Cholic acid (CA) | 25.00 | 16.67% |
| PVP | 2.50 | 1.66% |
| Eudragit® E PO | 5.50 | 3.67% |
| Maltitol Sweetpearl Roquette | 117.00 | 78.0% |
| **Total** | **150.00** | **100.0%** |

### Process of manufacturing of the pediatric formulation

363.64 g of cholic acid were mixed with 36.36 g of polyvinylpyrrolidone K30 and then granulated by wet granulation using a high shear impeller. Water was used as granulation liquid. Cholic acid granules were then dried in a fluidized bed dryer (Glatt GPCG1). 400 g of cholic acid granules were then coated with an aqueous solution of Eudragit® EPO in a Wurster fluidized bed dryer granulator to achieve a mass of 5.5 mg for 25 mg of cholic acid (corresponding to 80 g of dry Eudragit® EP O). Spraying rate was of 6-7 g/min was applied and parameters were adjusted to ensure the product temperature was comprised between 25 and 30°C. Coated cholic acid granules were then dried and parameters were adjusted to ensure the temperature of the product to be not more than 40°C.

Finally, the powder was mixed with qs 100% of maltitol Sweetpearl Roquette.

### Example 2: Manufacturing of the cholic acid pediatric granules with granulation with an excipient, then coating.

### Formulation 2a

The following table presents the formulation of the cholic acid pediatric granules.

| **Chemical name ingredient** | **Dosage unit (mg)** | **Quantity %(w/w)** |
|---|---|---|
| Cholic acid (CA) | 25.00 | 16.67% |
| Dicalcium phosphate | 87.50 | 58.33% |
| PVP | 11.25 | 7.50% |
| Eudragit® E PO | 26.25 | 17.50% |
| **Total** | **150.00** | **100.0%** |

### Process of manufacturing of the pediatric granules

80.7 g of cholic acid were mixed with 36.3 g of polyvinylpyrrolidone and 282 g of dicalcium phosphate and then granulated by wet granulation using a high shear impeller. Water was used as granulation liquid. Cholic acid granules were then dried in a fluidized bed dryer (Glatt GPCG1). 400 g of Cholic acid granules were then coated with an aqueous solution of Eudragit® EPO in a Wurster fluidized bed dryer granulator to achieve a mass of 26.25 mg for 25 mg of Cholic acid (corresponding to about 84 g of dry Eudragit® EP O). Spraying rate was of 6-7 g/min was applied and parameters were adjusted to ensure the product temperature was comprised between 25 and 30°C. Coated cholic acid granules were then dried and parameters were adjusted to ensure the temperature of the product to be not more than 40°C.

### Formulation 2b

The following table presents another formulation of the cholic acid pediatric granules.

| **Chemical name ingredient** | **Dosage unit (mg)** | **Quantity %(w/w)** |
|---|---|---|
| Cholic acid (CA) | 25.00 | 38.05% |
| Mannitol | 10.00 | 15.22% |
| Microcristalline cellulose | 10.00 | 15.22% |
| HPC | 1.40 | 2.13% |
| Eudragit® E PO | 19.30 | 29.38% |
| **Total** | **65.70** | **100.0%** |

### Process of manufacturing of the pediatric granules

25 g of cholic acid were mixed with 1.4 g of HPC, 10 g of mannitol and 10 g of microcrystalline cellulose and then granulated by wet granulation using a high shear impeller. Water was used as granulation liquid. Cholic acid granules were then dried in a fluidized bed dryer (Glatt GPCG1). 46.4 g of Cholic acid granules were then coated with an aqueous solution of Eudragit® EPO in a Wurster fluidized bed dryer granulator to achieve a mass of 19.30 mg for 25 mg of Cholic acid. Spraying rate was of 6-7 g/min was applied and parameters were adjusted to ensure the product temperature was comprised between 25 and 30°C. Coated cholic acid granules were then dried and parameters were adjusted to ensure the temperature of the product to be not more than 40°C.

### Example 3: Manufacturing of the cholic acid pediatric formulation with granulation with excipients, then coating and tabletting.

### Formulation 3a

| **Chemical name ingredient** | **Dosage unit (mg)** | **Quantity %(w/w)** |
|---|---|---|
| Cholic acid (CA) | 25.00 | 16.67% |
| Mannitol | 10 | 6.67% |
| Microcrystalline cellulose | 10 | 6.67% |
| HPC | 1.40 | 0.93% |
| Eudragit® E PO | 24.14 | 16.09% |
| Microcrystalline cellulose | 72.71 | 48.47% |
| Sodium croscarmellose | 6.00 | 4.00% |
| Magnesium stearate | 0.75 | 0.50% |
| **Total** | **150.00** | **100.00%** |

### Formulation 3b

| **Chemical name ingredient** | **Dosage unit (mg)** | **Quantity %(w/w)** |
|---|---|---|
| Cholic acid (CA) | 25.00 | 16.67% |
| Mannitol | 10 | 6.67% |
| Microcrystalline cellulose | 10 | 6.67% |
| HPC | 1.40 | 0.93% |
| Eudragit® E PO | 19.30 | 12.87% |
| Microcrystalline cellulose | 77.55 | 51.70% |
| Sodium croscarmellose | 6.00 | 4.00% |
| Sodium stearyl fumarate | 0.75 | 0.50% |
| **Total** | **150.00** | **100.00%** |

### Process of manufacturing of the pediatric formulation

377.16 g of cholic acid was mixed with 21.12 g of HPC, 150.86 g of microcrystalline cellulose and 150.86 g of mannitol and then granulated by wet granulation using a high shear impeller. Water was used as granulation liquid. Cholic acid granules were then dried in a fluidized bed dryer (Retsch).

628.84 g of the prepared cholic acid granules were then coated with an aqueous solution of Eudragit® EPO in a Wurster fluidized bed dryer granulator to achieve a mass of 24.14 mg for 25 mg of cholic acid (corresponding to 327.16 g of dry Eudragit® EP O). Spraying rate was of 8-10 g/min was applied and parameters were adjusted to ensure the product temperature was comprised between 25 and 35°C. Coated cholic acid granules were then dried and parameters were adjusted to ensure the temperature of the product to be not more than 40°C.

117.57 g of the coated cholic acid granules were mixed with 121.18 g of microcrystalline cellulose and 10.00 g of sodium croscarmellose and blended during 5 minutes at 2 x 22 rpm. 1.25 g of magnesium stearate or sodium stearyl fumarate was then added and the mixture was blended during 2 minutes at 2 x 22 rpm. The final blend was then tabletted on a rotative press to obtain dispersible tablets.

### Example 4: Bitterness test.

### Formulation 4 : EthylCellulose

| **Chemical name ingredient** | **Dosage unit (mg)** | **Quantity %(w/w)** |
|---|---|---|
| Cholic acid (CA) | 25.00 | 81.7% |
| PVP | 2.17 | 7.1% |
| Ethylcellulose+ Hypromellose 2910 | 3.43 | 11.2% |
| **Total** | **30.60** | **100.0%** |

### Formulation 5: Eudragit® RL30D + Carboxymethylcellulose sodique (Na-CMC)

| **Chemical name ingredient** | **Dosage unit (mg)** | **Quantity %(w/w)** |
|---|---|---|
| Cholic acid (CA) | 25.00 | 81.94% |
| PVP | 2.17 | 7.13% |
| Eudragit® RL 30D + Na-CMC + Polysorbate 80 + Talc | 3.33 | 10.93% |
| **Total** | **30.50** | **100.0%** |

### Formulation 6: Eudragit® E PO

| **Chemical name ingredient** | **Dosage unit (mg)** | **Quantity %(w/w)** |
|---|---|---|
| Cholic acid (CA) | 25.00 | 81.94% |
| PVP | 2.17 | 7.13% |
| Eudragit® E PO | 3.33 | 10.93% |
| **Total** | **30.50** | **100.0%** |

### Process of manufacturing of the pediatric formulation

Cholic acid was mixed with polyvinylpyrrolidone K30 granulated by wet granulation using a Top Spray fluidized bed dryer granulator (Glatt GPCG1). Water was used as granulation liquid. Spraying rate was of 10-12 g/min was applied and parameters were adjusted to ensure the product temperature was comprised between 25 and 35°C. Coated cholic acid granules were then dried and parameters were adjusted to ensure the temperature of the product to be not more than 40°C.

Cholic acid granules were then coated with a taste-masking compound in a Wurster fluidized bed dryer granulator. Spraying rate of 6-7 g/min was applied and parameters were adjusted to ensure the product temperature was comprised between 25 and 45°C. Coated cholic acid granules were then dried and parameters were adjusted to ensure the temperature of the product to be not more than 45°C.

### Results

The formulations 1-2-3-4-5-6 were then swallowed as it is, or/and in suspension in water to evaluate their bitterness. The rate of emergence of the bitterness and its intensity were then evaluated for each formulation by a panel of 5 persons. A score between 0 and ++ was assigned. ++ means that the formulation is highly bitter and 0 means there is no bitterness.

| **Formulation** | **Intensity** | **Rate of emergence of the bitterness** | |
|---|---|---|---|
| | | **Dry** | **Suspension in water** |
| Formulation 1 | 0 | - | No bitter taste |
| Formulation 2a | 0 | - | No bitter taste |
| Formulation 2b | 0 | - | No bitter taste |
| Formulation 3a | 0 | - | No bitter taste |
| Formulation 3b | 0 | - | No bitter taste |
| Formulation 4 | ++ | 0-10 seconds | ∼10 seconds |
| Formulation 5 | ++ | 0-10 seconds | ∼10 seconds |
| Formulation 6 | + | 0-10 seconds | ∼10 seconds |

## Claims

1. Granule comprising a core, said core comprising at least one unbuffered primary bile acid; at least one binder; at least one excipient, referred to as Excipient A, preferably selected from sugars, polyols and polymers; and a coating surrounding the core, said coating comprising at least one gastric-fluid-soluble-and-taste-masking compound.

2. Granule according to claim **1,** wherein the unbuffered primary bile acid is unbuffered cholic acid.

3. Granule according to claim **1** or claim **2,** wherein the at least one gastric-fluid-soluble-and-taste-masking compound is selected from starches, polyvinylpyrrolidones, gelatin, methylcellulose, hydroxyethylcellulose, hydroxypropylcellulose, hydroxypropyl methyl cellulose, microcrystalline cellulose and methacrylic acid copolymer and combinations thereof.

4. Granule according to anyone of claims **1** to **3,** wherein the amount of said taste-masking compound ranges from 1% w/w to 90% w/w, preferably from 5% w/w to 50% w/w, more preferably from 10% w/w to 40% w/w, in weight to the total weight of said granule.

5. Granule according to anyone of claims **1** to **4,** wherein the amount of said excipient A ranges from 0% w/w to 70% w/w, preferably from 0% w/w to 60% w/w, more preferably from 0% w/w to 40% w/w, in weight to the total weight of said granule.

6. Granule according to anyone of claims **1** to **5,** comprising:
- from 30% to 50% in weight relative to the total weight of the granule of cholic acid;
- from 10% to 20% in weight relative to the total weight of the formulation of mannitol;
- from 10% to 20% in weight relative to the total weight of the formulation of microcrystalline cellulose;
- from 0.5% to 5% in weight relative to the total weight of the formulation of polyvinylpyrrolidone;
- from 20% to 40% in weight relative to the total weight of the formulation of Poly (butyl methacrylate-co-(2-dimethylaminoethyl) methacrylate-co-methyl methacrylate) (1:2:1).

7. Formulation comprising at least one granule according to anyone of claims **1** to **6,** wherein the formulation further comprises at least one excipient B, selected from at least one diluent, at least one disintegrant or a combination thereof; preferably the at least one excipient B is selected from sugars, polyols, natural polymer, synthetic polymer, hydrophobic or hydrophilic lubricants.

8. Formulation according to claim **7,** wherein the particle size distribution of said at least one unbuffered primary bile acid granules in the formulation ranges from 30 µm to 1 000 µm, preferably from 30 µm to 710 µm, more preferably from 90 µm to 500 µm, even more preferably from 125 µm to 500 µm.

9. Formulation according to claim **7** or claim **8,** comprising:
- from 10% to 20% in weight relative to the total weight of the formulation of cholic acid;
- from 1% to 12% in weight relative to the total weight of the formulation of Mannitol;
- from 1% to 12% in weight relative to the total weight of the formulation of Microcrystalline cellulose;
- from 0.01% to 1.5% in weight relative to the total weight of the formulation of hydroxylpropylcellulose;
- from 8% to 18% in weight relative to the total weight of the formulation of Poly (butyl methacrylate-co-(2-dimethylaminoethyl) methacrylate-co-methyl methacrylate) (1:2:1);
- from 43% to 53% in weight relative to the total weight of the formulation of microcrystalline cellulose;
- from 0% to 10% in weight relative to the total weight of the formulation of sodium croscarmellose;
- from 0% to 10% in weight relative to the total weight of the formulation of magnesium stearate.

10. Process for the manufacturing a formulation according to anyone of claims **7** to **9,** wherein the process consists of:
a) Mixing at least one unbuffered primary bile acid, especially unbuffered cholic acid with at least one excipient A;
b) Granulating the said granule containing at least one unbuffered primary bile acid, especially unbuffered cholic acid with the at least one excipient A using a wet granulation in presence of at least one binder;
c) Drying the said at least one unbuffered primary bile acid granules, especially unbuffered cholic acid granules;
d) Calibrating the said at least one unbuffered primary bile acid granules, especially unbuffered cholic acid granules;
e) Coating the said at least one unbuffered primary bile acid granules, especially unbuffered cholic acid granules with at least one gastric-fluid-soluble-and-taste-masking compound;
f) Drying the said coated at least one unbuffered primary bile acid granules, especially coated unbuffered cholic acid granules;
g) Calibrating the said coated at least one unbuffered primary bile acid granules, especially coated unbuffered cholic acid granules;
h) Mixing at least one coated unbuffered primary bile acid granule, especially at least one coated unbuffered cholic acid granule, with at least one excipient B; and
i) Tableting the final blend to obtain dispersible or orodispersible tablets or filling the final blend in sprinkle capsules or sachets.

11. Pharmaceutical composition comprising at least one granule according to anyone of claims **1** to **6** or a formulation according to anyone of claims **7** to **9.**

12. Medicament comprising at least one granule according to anyone of claims **1** to **6** or a formulation according to anyone of claims **7** to **9** or a pharmaceutical composition according to claim **11.**

13. Unit dosage form comprising at least one granule according to anyone of claims **1** to **6** or a formulation according to anyone of claims **7** to **9** or a pharmaceutical composition according to claim **11.**

14. Formulation according to anyone of claims **7** to **9,** or pharmaceutical composition according to claim **11,** for use in the treatment of a patient suffering from a deficiency in primary bile acid synthesis.

15. The formulation or the pharmaceutical composition for use according to claim **14,** wherein the patient is aged from 1 month to 6 years old.

## Patentansprüche

1. Körnchen, umfassend einen Kern, wobei der Kern mindestens eine ungepufferte primäre Gallensäure umfasst; mindestens ein Bindemittel; mindestens einen Trägerstoff, bezeichnet als Trägerstoff A, vorzugsweise ausgewählt aus Zuckern, Polyolen und Polymeren; und eine Beschichtung, die den Kern umgibt, wobei die Beschichtung mindestens eine magenflüssigkeitslösliche- und - geschmackmaskierende Verbindung umfasst.

2. Körnchen nach Anspruch **1,** wobei die ungepufferte primäre Gallensäure ungepufferte Cholsäure ist.

3. Körnchen nach Anspruch **1** oder Anspruch **2,** wobei die mindestens eine magenflüssigkeitslösliche- und -geschmackmaskierende Verbindung ausgewählt ist aus Stärken, Polyvinylpyrrolidonen, Gelatin, Methylcellulose, Hydroxyethylcellulose, Hydroxypropylcellulose, Hydroxypropylmethylcellulose, mikrokristalliner Cellulose und Methacrylsäurecopolymer und Kombinationen daraus.

4. Körnchen nach einem der Ansprüche **1** bis **3,** wobei die Menge der geschmackmaskierenden Verbindung im Bereich von 1 % w/w bis 90 % w/w, vorzugsweise von 5 % w/w bis 50 % w/w, insbesondere bevorzugt von 10 % w/w bis 40 % w/w Gewicht des Gesamtgewichts des Körnchens liegt.

5. Körnchen nach einem der Ansprüche **1** bis **4,** wobei die Menge des Trägerstoffs A im Bereich von 0 % w/w bis 70 % w/w, vorzugsweise von 0 % w/w bis 60 % w/w, insbesondere bevorzugt von 0 % w/w bis 40 % w/w Gewicht des Gesamtgewichts des Körnchens liegt.

6. Körnchen nach einem der Ansprüche **1** bis **5,** umfassend:
- von 30 bis 50 Gew% Cholsäure mit Bezug auf das Gesamtgewicht des Körnchens;
- von 10 bis 20 Gew% Mannitol mit Bezug auf das Gesamtgewicht der Formulierung;
- von 10 bis 20 Gew% mikrokristalline Cellulose mit Bezug auf das Gesamtgewicht der Formulierung;
- von 0,5 % bis 5 Gew% Polyvinylpyrrolidon mit Bezug auf das Gesamtgewicht der Formulierung;
- von 20 bis 40 Gew% Poly(butylmethacrylat-co-(2-dimethylaminoethyl)methacrylat-co-methylmethacrylat) (1:2:1) mit Bezug auf das Gesamtgewicht der Formulierung.

7. Formulierung, umfassend mindestens ein Körnchen nach einem der Ansprüche **1** bis **6,** wobei die Formulierung weiter mindestens einen Trägerstoff B umfasst, ausgewählt aus mindestens einem Verdünnungsmittel, mindestens einem Zerfallsmittel oder einer Kombination davon; vorzugsweise der mindestens eine Trägerstoff B ausgewählt ist aus Zuckern, Polyolen, natürlichem Polymer, synthetischem Polymer, hydrophoben oder hydrophilen Schmiermitteln.

8. Formulierung nach Anspruch **7,** wobei die Partikelgrößenverteilung der mindestens einen ungepufferten primären Gallensäurekörnchen in der Formulierung im Bereich von 30 µm bis 1000 µm liegt, vorzugsweise im Bereich von 30 µm bis 710 µm, insbesondere bevorzugt im Bereich von 90 µm bis 500 µm, noch bevorzugter im Bereich von 125 µm bis 500 µm.

9. Formulierung nach Anspruch **7** oder Anspruch **8,** umfassend:
- von 10 bis 20 Gew% Cholsäure mit Bezug auf das Gesamtgewicht der Formulierung;
- von 1 bis 12 Gew% Mannitol mit Bezug auf das Gesamtgewicht der Formulierung;
- von 1 bis 12 Gew% mikrokristalline Cellulose mit Bezug auf das Gesamtgewicht der Formulierung;
- von 0,01 bis 1,5 Gew% Hydroxylpropylcellulose mit Bezug auf das Gesamtgewicht der Formulierung;
- von 8 bis 18 Gew% Poly(butylmethacrylat-co-(2-dimethylaminoethyl)methacrylat-co-methylmethacrylat) (1:2:1) mit Bezug auf das Gesamtgewicht der Formulierung;
- von 43 bis 53 Gew% mikrokristalliner Cellulose mit Bezug auf das Gesamtgewicht der Formulierung;
- von 0 bis 10 Gew% Natriumcroscarmellose mit Bezug auf das Gesamtgewicht der Formulierung;
- von 0 bis 10 Gew% Magnesiumstearat mit Bezug auf das Gesamtgewicht der Formulierung.

10. Verfahren zur Herstellung einer Formulierung nach einem der Ansprüche **7** bis **9,** wobei das Verfahren aus Folgendem besteht:
a) Mischen von mindestens einer ungepufferten primären Gallensäure, insbesondere einer ungepufferten Cholinsäure, mit mindestens einem Trägerstoff A;
b) Granulieren des Körnchens, enthaltend mindestens eine ungepufferte primäre Gallensäure, insbesondere eine ungepufferte Cholsäure, mit dem mindestens einen Trägerstoff A, unter Verwendung einer nassen Granulierung in Anwesenheit von mindestens einem Bindemittel;
c) Trocknen der mindestens einen ungepufferten primären Gallensäurekörnchen, insbesondere ungepufferten Cholsäurekörnchen;
d) Kalibrieren der mindestens einen ungepufferten primären Gallensäurekörnchen, insbesondere ungepufferten Cholsäurekörnchen;
e) Beschichten der mindestens einen ungepufferten primären Gallensäurekörnchen, insbesondere ungepufferten Cholsäurekörnchen, mit mindestens einer magenflüssigkeitslöslichen- und -geschmackmaskierenden Verbindung;
f) Trocknen der mindestens einen ungepufferten primären Gallensäurekörnchen, insbesondere beschichteten ungepufferten Cholsäurekörnchen;
g) Kalibrieren der beschichteten mindestens einen ungepufferten primären Gallensäurekörnchen, insbesondere beschichteten ungepufferten Cholsäurekörnchen;
h) Mischen von mindestens einem ungepufferten primären Gallensäurekörnchen, insbesondere mindestens einem beschichteten ungepufferten Cholsäurekörnchen, mit mindestens einem Trägerstoff B; und
i) Tablettieren der endgültigen Mischung, um dispergierbare oder Schmelztabletten zu erhalten oder die endgültige Mischung in Streukapseln oder Beuteln zu füllen.

11. Pharmazeutische Zusammensetzung, umfassend mindestens ein Körnchen nach einem der Ansprüche **1** bis **6** oder eine Formulierung nach einem der Ansprüche **7** bis **9.**

12. Medikament, umfassend mindestens ein Körnchen nach einem der Ansprüche **1** bis **6** oder eine Formulierung nach einem der Ansprüche **7** bis **9** oder eine pharmazeutische Zusammensetzung nach Anspruch **11.**

13. Einheitsdosierungsform, umfassend mindestens ein Körnchen nach einem der Ansprüche **1** bis **6** oder eine Formulierung nach einem der Ansprüche **7** bis **9** oder eine pharmazeutische Zusammensetzung nach Anspruch **11.**

14. Formulierung nach einem der Ansprüche **7** bis **9** oder pharmazeutische Zusammensetzung nach Anspruch **11** zur Verwendung bei der Behandlung eines Patienten, der an einer Defizienz der Synthese von primärer Gallensäure leidet.

15. Formulierung oder pharmazeutische Zusammensetzung zur Verwendung nach Anspruch **14,** wobei der Patient im Alter von 1 Monat bis 6 Jahren ist.

## Revendications

1. Granulé comprenant un noyau, ledit noyau comprenant au moins un acide biliaire primaire non-tamponné ; au moins un liant, au moins un excipient, référencée en tant qu'Excipient A, de préférence étant sélectionné parmi des sucres, des polyols et des polymères ; et un enrobage enrobant ledit noyau, ledit enrobage comprenant au moins un composé soluble dans les fluides gastriques et masquant le goût.

2. Granulé selon la revendication **1,** dans lequel l'acide biliaire primaire non-tamponné est l'acide cholique non-tamponné.

3. Granulé selon la revendication **1** ou la revendication **2,** dans lequel l' au moins un composé soluble dans les fluides gastriques et masquant le goût est sélectionné parmi des amidons, des polyvinylpyrrolidones, la gélatine, la méthylcellulose, l'hydroxyéthylcellulose, l'hydroxypropylcellulose, l'hydroxypropyl méthyl cellulose, la cellulose microcristalline, un copolymère d'acide méthacrylique et leurs combinaisons.

4. Granulé selon l'une quelconque des revendications **1** à **3,** dans lequel la quantité dudit composé soluble dans les fluides gastriques et masquant le goût varie de 1% p/p à 90% p/p, de préférence de 5% p/p à 50% p/p, de manière encore plus préférentielle de 10% p/p à 40% p/p, en poids par rapport au poids total dudit granulé.

5. Granulé selon l'une quelconque des revendications **1** à **4,** dans lequel la quantité dudit Excipient A varie de 0% p/p à 70% p/p, de préférence de 0% p/p à 60% p/p, de manière encore plus préférentielle de 0% p/p à 40% p/p, en poids par rapport au poids total dudit granulé.

6. Granulé selon l'une quelconque des revendications **1** à **5,** comprenant :
- de 30% p/p à 50% p/p en poids d'acide cholique par rapport au poids total dudit granulé ;
- de 10% p/p à 20% p/p en poids de mannitol par rapport au poids total de la formulation ;
- de 10% p/p à 20% p/p en poids de cellulose microcristalline par rapport au poids total de la formulation ;
- de 0,5% p/p à 5% p/p en poids de polyvinylpyrrolidone par rapport au poids total de la formulation ;
- de 20% p/p à 40% p/p en poids de Poly(butyl méthacrylate-co-(2-diméthylaminoéthyl) méthacrylate- co- méthyl méthacrylate) (1:2:1) par rapport au poids total de la formulation.

7. Formulation comprenant au moins un granulé selon l'une quelconque des revendications **1** à **6,** ladite formulation comprenant en outre au moins un Excipient B, choisi parmi au moins un diluant, au moins un désintégrant ou leur combinaison, de préférence ledit au moins un Excipient B est sélectionné parmi des sucres, des polyols, des polymères naturels, des polymères synthétiques, des lubrifiants hydrophobes ou hydrophiles.

8. Formulation selon la revendication **7,** dans laquelle la distribution de la taille des particules dudit au moins un granulé d'acide biliaire primaire non-tamponné dans la formulation est de 30 µm à 1 000 µm, de préférence de 30 µm à 710 µm, de manière préférentielle de 90 µm à 500 µm, de manière encore plus préférentielle de 125 µm à 500 µm.

9. Formulation selon la revendication **7** ou la revendication **8,** comprenant :
- de 10% p/p à 20% p/p en poids d'acide cholique par rapport au poids total de la formulation ;
- de 1% p/p à 12% p/p en poids de mannitol par rapport au poids total de la formulation ;
- de 1% p/p à 12% p/p en poids de cellulose microcristalline par rapport au poids total de la formulation ;
- de 0,01% p/p à 1,5% p/p en poids d'hydroxypropylcellulose par rapport au poids total de la formulation ;
- de 8% p/p à 18% p/p en poids de Poly(butyl méthacrylate-co-(2-diméthylaminoéthyl) méthacrylate- co- méthyl méthacrylate) (1:2:1) par rapport au poids total de la formulation.
- de 43% p/p à 53% p/p en poids de cellulose microcristalline par rapport au poids total de la formulation;
- de 0% p/p à 10% p/p en poids de croscarmellose sodique par rapport au poids total de la formulation;
- de 0% p/p à 10% p/p en poids de stéarate de magnésium par rapport au poids total de la formulation.

10. Procédé pour la préparation de la formulation selon l'une quelconque des revendications **7** à **9,** ledit procédé consistant en :
a) Mélanger au moins un acide biliaire primaire non-tamponné, notamment de l'acide cholique non-tamponné avec au moins un Excipient A;
b) Granuler ledit granule comprenant au moins un acide biliaire primaire non-tamponné, notamment de l'acide cholique non-tamponné, avec au moins un Excipient A en utilisant de la granulation humide en présence d'au moins un liant ;
c) Sécher ledit au moins un granulé d'acide biliaire primaire non-tamponné, notamment un granulé d'acide cholique non-tamponné ;
d) Calibrer ledit au moins un granulé d'acide biliaire primaire non-tamponné, notamment un granulé d'acide cholique non-tamponné ;
e) Enrober ledit au moins un granulé d'acide biliaire primaire non-tamponné, notamment un granulé d'acide cholique non-tamponné, avec au moins un composé soluble dans les fluides gastriques et masquant le goût
f) Sécher ledit au moins un granulé d'acide biliaire primaire non-tamponné enrobé, notamment un granulé d'acide cholique non-tamponné enrobé ;
g) Calibrer ledit au moins un granulé d'acide biliaire primaire non-tamponné enrobé, notamment un granulé d'acide cholique non-tamponné enrobé
h) Mélanger l'au moins un granulé d'acide biliaire primaire non-tamponné, notamment un granulé d'acide cholique non-tamponné enrobé, avec au moins un Excipient B ; et
i) Compresser le mélange final afin d'obtenir des comprimés dispersibles ou orodispersibles, ou remplir des sachets ou des capsules à saupoudrer du mélange final.

11. Composition pharmaceutique comprenant au moins un granule selon l'une quelconque des revendications **1** à **6** ou une formulation selon l'une quelconque des revendications **7** à **9.**

12. Médicament comprenant au moins un granule selon l'une quelconque des revendications **1** à **6** ou la composition pharmaceutique selon la revendication **11.**

13. Dose unitaire comprenant au moins un granule selon l'une quelconque des revendications **1** à **6,** une formulation selon l'une quelconque des revendications **7** à **9** ou la composition pharmaceutique selon la revendication **11.**

14. Formulation selon l'une quelconque des revendications **7** à **9,** ou composition pharmaceutique selon la revendication **11,** pour son utilisation dans le traitement d'un patient souffrant d'une déficience de synthèse d'acide biliaire primaire.

15. La formulation ou la composition pharmaceutique selon la revendication **14,** dans laquelle le patient est âgé d'1 mois à 6 ans.
